Europäisches Patentamt

European Patent Office

Office européen des brevets

(19)

(11) Veröffentlichungsnummer: **0 289 847**
**B1**

(12) EUROPÄISCHE PATENTSCHRIFT

(45) Veröffentlichungstag der Patentschrift:
31.10.90

(51) Int. Cl.⁵: **C07C 309/86**

(21) Anmeldenummer: 88106181.6

(22) Anmeldetag: 19.04.88

(54) Verfahren zur Herstellung von 2-Chlor-5-nitro- und 4-Chlor-3-nitrobenzolsulfonsäurechlorid.

(30) Priorität: 01.05.87 DE 3714611
02.10.87 DE 3733290

(43) Veröffentlichungstag der Anmeldung:
09.11.88 Patentblatt 88/45

(45) Bekanntmachung des Hinweises auf die Patenterteilung:
31.10.90 Patentblatt 90/44

(84) Benannte Vertragsstaaten:
CH DE FR GB LI

(56) Entgegenhaltungen:
EP-B- 0 001 275
EP-B- 0 001 276
DE-A- 2 635 279
DE-A- 2 635 281
DE-A- 2 721 429
US-A- 2 511 547

(73) Patentinhaber: BAYER AG,
D-5090 Leverkusen 1 Bayerwerk(DE)

(72) Erfinder: Horstmann, Walter, Dr., Eichenweg 17,
D-5060 Bergisch Gladbach 2(DE)

**Beschreibung**

Die vorliegende Erfindung betrifft ein Verfahren zur Herstellung von 2-Chlor-5-nitro-benzolsulfonsäurechlorid (I) und 4-Chlor-3-nitrobenzolsulfonsäurechlorid (II).

Für die Herstellung von (I) und (II) sind verschiedene Verfahren beschrieben.

Ein Verfahren zur Herstellung von (I) besteht darin, daß man p-Chlornitrobenzol mit Chlorsulfonsäure im Molverhältnis 1:5 bei 120-130°C umsetzt und das erhaltene Rohprodukt aus Tetrachlorkohlenstoff umkristallisiert (A.R. Goldfarb und B. Berk, Am. Soc. 65 [1943] 738.

Die Autoren geben eine Ausbeute von 55 % der Theorie und einen Schmelzpunkt von 85-87°C an. Wegen der mäßigen Ausbeute und der erforderlichen Umkristallisation aus Tetrachlorkohlenstoff scheidet dieses Verfahren für die technische Herstellung von (I) aus.

Ein weiteres Verfahren zur Herstellung von (I) beruht darauf, daß man das Kaliumsalz der 2-Chlor-5-nitrobenzolsulfonsäure mit Phosphorpentachlorid zur Umsetzung bringt (P. Fischer. Ber. 24 [1891] 3196).

Durch Umkristallisieren aus Ether erhält der Autor das gesuchte Sulfonsäurechlorid mit einem Schmelzpunkt von 89-90°C. Dieses Verfahren ist ebenfalls von Nachteil, da man zunächst das trockene und pulversierte Kaliumsalz der 2-Chlor-5-nitro-benzolsulfonsäure gewinnen muß, also einen Verfahrensschritt mehr als bei direkter Chlorsulfonierung hat.

Die Herstellung von 4-Chlor-3-nitro-benzolsulfonsäurechlorid (II) beschreiben beispielsweise E.V. Zakharov und Mitarbeiter (Zhurnal Organicheskoi Khimii, Vol. 1, Nr. 10, pp 1866-1868 [1965]). Die Autoren setzen o-Chlornitrobenzol mit Chlorsulfonsäure im Molverhältnis 1:6 bei 140°C um und erhalten nach Destillation des Rohproduktes eine Ausbeute an (II) von 92,2 %, nach Umkristallisation aus Ether einen Schmelzpunkt von 61,1°C. Bei der Nacharbeitung der Herstellungsvorschrift fanden wir Ausbeuten von 73-79 % der Theorie (Schmelzpunkt ohne Reinigung: 57,0-57,5°C).

In dem US-Patent 884 206 ist die Herstellung von (II) durch Umsetzung des Kaliumsalzes der 4-Chlor-3-nitrobenzolsulfonsäure mit Phosphorpentachlorid und Phosphoroxychlorid beschrieben. Die Autoren erhalten mit dieser Methode zwar eine Ausbeute von 93 %, aber hier gilt, wie oben bereits für (I) dargelegt, daß die technische Herstellung wegen der vorherigen Gewinnung des trockenen und gemahlenen Kaliumsalzes der Sulfonsäure besonders kostenintensiv ist.

Es wurde nun überaschenderweise gefunden, daß man 2-Chlor-5-nitro- und 4-Chlor-3-nitro-benzolsulfonsäurechlorid (I und II) in guter Ausbeute und Qualität gewinnen kann, wenn man die bei der Umsetzung von o- und p-Chlornitrobenzol mit Oleum anfallenden, die entsprechenden Chlornitrobenzolsulfonsäuren enthaltenden Sulfiergemische direkt weiter mit Thionylchlorid und/oder Chlorsulfonsäure behandelt.

Beispielsweise erfolgt die Umsetzung von p-Chlornitrobenzol mit Oleum nach dem H.E. Fierz-David und L. Blangey (Grundlegende Operationen der Farbenchemie, 8. Auflage [1952]96) beschriebenen Verfahren:

"Man mischt 100 g p-Nitrochlorbenzol bei 50°C mit 100 g Schwefelsäuremonohydrat und läßt unter Rühren 280 g Oleum von 25 % SO₃-Gehalt einlaufen. Man erwärmt so lange auf 100-110°C, bis das Nitrochlorbenzol verschwunden ist".

Das so gewonnene Sulfiergemisch, das 31,5 %ig an 2-Chlor-5-nitrobenzolsulfonsäure und 4,0 %ig an SO₃ ist, kann direkt als Einsatzprodukt zur Herstellung von 2-Chlor-5-nitro-benzolsulfonsäurechlorid verwendet werden.

In analoger Weise verfährt man bei der Herstellung eines Sulfiergemisches, das 4-Chlor-3-nitrobenzolsulfonsäure zur direkten Weiterverarbeitung auf (II) enthält.

Zur Überführung der in den fertigen Sulfiergemischen enthaltenen Chlornitro-benzolsulfonsäuren in die entsprechenden Sulfonsäurechloride (I) und (II) eignen sich besonders Thionylchlorid, Chlorsulfonsäure und Gemische dieser Chlorierungsmittel, die man in der Regel im Überschuß, bezogen auf die Chlornitro-benzolsulfonsäure, zum Einsatz bringt.

Beim Arbeiten mit Thionylchlorid erzielt man bereits sehr gute Ausbeuten, wenn man ein Molverhältnis von Chlornitro-benzolsulfonsäure: Thionylchlorid = 1:2 - 1:10 vorzugsweise 1:3 - 1:5 wählt, bei Verwendung von Chlorsulfonsäure ist ein größerer Überschuß zweckmäßig, z.B. 1:5 - 1:15 vorzugsweise 1:8 - 1:10.

Das Verfahren kann auch in der Weise durchgeführt werden, daß man nacheinander 2-10 Mol, vorzugsweise 4-6 Mol Chlorsulfonsäure und 1-5 Mol, vorzugsweise 1-3 Mol Thionylchlorid pro Mol Chlornitrobenzolsulfonsäure zur Anwendung bringt.

Bei der Durchführung der Umsetzung kann man das Sulfiergemisch vorlegen und das Chlorierungsmittel eindosieren, die umgekehrte Arbeitsweise ist aber auch möglich. Man arbeitet bei Temperaturen zwischen 40 und 120°C und vorzugsweise drucklos. Beim Chlorieren mit Thionylchlorid legt man beispielsweise das Sulfiergemisch vor, setzt Thionylchlorid bei 40-50°C zu und erhitzt zur Vervollständigung der Umsetzung noch eine zeitlang auf 80-100°C.

Zur Erzielung einer guten Ausbeute ist der Gehalt des einzusetzenden Sulfiergemisches an Oleum ebenfalls von Bedeutung. Gehalte von 4-6 % Oleum haben sich im Falle der Herstellung von (I) als besonders günstig erwiesen, aber auch niedrigere Gehalte führen zu guten Ausbeuten.

Die resultierenden Reaktionsgemische arbeitet man in üblicher Weise auf: Man kühlt auf Raumtemperatur ab und rührt in Eiswasser ein, wobei sich die Chlornitrobenzolsulfonsäurechloride als beige gefärbte Produkte in fester, gut filtrierbarer Form abscheiden. Nach Filtration und gründlichem Auswaschen der Schwefelsäure erhält man Verbindungen, die ausreichend rein zur Weiterverarbeitung sind. An Verunreinigungen enthalten sie Spuren der entsprechenden Sulfonsäuren sowie noch ca. 0,5 % der Bis-(chlornitrophenyl)-sulfone.

2-Chlor-5-nitro- und 4-Chlor-3-nitro-benzolsulfonsäurechlorid sind aufgrund ihrer funktionellen Gruppen interessante Vorprodukte für zahlreiche Synthese, z.B. zur Herstellung von Sulfonamiden und Sulfinsäuren. Die letzteren finden neuerdings zur Herstellung von Farbstoffen der Triphendioxazin-Reihe (vergl. EP-A 0 153 599) Verwendung.

Beispiel 1

496 g eines Sulfiergemisches (aus der Umsetzung von p-Nitrochlorbenzol in Schwefelsäuremonohydrat mit 25 %igem Oleum bei etwa 100-110°C), das 237,5 g (1,0 Mol) 2-Chlor-5-nitro-benzolsulfonsäure und 5 % Schwefeltrioxid enthält, werden bei 45°C vorgelegt und im Verlaufe von 1,5 Stunden 595 g (5,0 Mol) Thionylchlorid bei 45-50°C unter gelindem Erwärmen von außen zugetropft. Man rührt 1 Stunde bei dieser Temperatur nach, heizt auf 100 ± 2°C auf und hält diese Temperatur 2 Stunden lang.

Nach dem Abkühlen auf Raumtemperatur rührt man das Reaktionsgemisch in 3100 ml Eiswasser ein, so daß die Innentemperatur von 12°C nicht überschritten wird. Das ausgefallene Produkt wird abfiltriert und mit Wasser säurefrei gewaschen.

Man erhält 320 g feuchtes Produkt, das 236 g 2-Chlor-5-nitro-benzolsulfonsäurechlorid enthält, entsprechend einer Ausbeute von 92 % der Theorie. An Nebenprodukt werden 0,3 % Bis-(2-chlor-5-nitrophenyl)-sulfon gefunden.

Da bei der Trocknung des feuchten Säurechlorids mit Hydrolyse zu rechnen ist, verarbeitet man es nach Möglichkeit in feuchter Form, z.B. bei der Herstellung von 2-Chlor-5-nitro-benzolsulfinsäure, einem Vorprodukt für wertvolle Triphendioxazinfarbstoffe.

Nach dem Umkristallisieren einer getrockneten Probe aus Diethylether (Aktivkohle) findet man einen Schmelzpunkt von 90-91°C. Die dünnschichtchromatographische Untersuchung (DC-Fertigplatten der Firma Merck, Kieselgel 60, F 254) des Produkts unter Verwendung des Laufmittelgemisches Methylethylketon/Diethylamin/Ammoniak (10 %ig) = 100/20/20 ergibt einen $R_f$-Wert von 0,95.

Beispiel 2

1165 g (10 Mol) Chlorsulfonsäure werden bei 85°C vorgelegt und 496 g Sulfiergemisch (siehe Beispiel 1), das 237,5 g (1,0 Mol) 2-Chlor-5-nitro-benzolsulfonsäure und 5 % Schwefeltrioxid enthält und 70°C warm ist, bei 85 ± 2°C im Verlaufe einer Stunde zugetropft. Anschließend erhöht man die Temperatur auf 110 ± 2°C und hält diese Temperatur zwei Stunden lang.

Nach dem Abkühlen des Reaktionsgemisches auf 20-25°C trägt man auf 3 kg Eiswasser aus, so daß die Temperatur 12°C nicht überschreitet. Nach Filtration und Auswaschen der Schwefelsäure erhält man 265 g feuchtes Produkt, das 187 g 2-Chlor-5-nitro-benzolsulfonsäurechlorid, entsprechend 73 % der Theorie, und 0,1 % Bis-(2-chlor-5-nitrophenyl)-sulfon enthält.

Beispiel 3

450,8 g eines Sulfiergemisches (aus der Umsetzung von o-Nitrochlorbenzol in Schwefelsäuremonohydrat mit 25 %igem Oleum bei etwa 100-110°C), das 237,5 g (1,0 Mol) 4-Chlor-3-nitro-benzolsulfonsäure, 5,9 g Schwefeltrioxid und 207,4 g Schwefelsäure enthält, wird bei 45°C vorgelegt und im Verlaufe von 2 Stunden (nach Maßgabe der Gasentwicklung von Salzsäure und Schwefeldioxid) mit 357 g (3,0 Mol) Thionylchlorid bei 45-50°C unter gelindem Erwärmen von außen versetzt. Man rührt 1 Stunde bei dieser Temperatur nach, heizt auf 70 ± 2°C und hält diese Temperatur 2 Stunden lang.

Nach dem Abkühlen auf 20-25°C trägt man das Reaktionsgemisch auf ca. 3 l Eiswasser aus, so daß die Temperatur 5°C nicht überschreitet. Das ausgefallene beigefarbene Produkt wird abgesaugt und mit kaltem Wasser säurefrei gewaschen.

Man erhält 318 g feuchtes Produkt, das 75,7 %ig an 4-Chlor-3-nitro-benzolsulfonsäurechlorid ist, entsprechend einer Ausbeute von 94,0 % der Theorie. An Verunreinigungen werden je 0,1 % Bis-(4-Chlor-3-nitrophenyl)-sulfon und 4-Chlor-3-nitro-benzolsulfonsäure gefunden.

Das Rohprodukt schmilzt bei 58-59°C und ist ausreichend rein für weitere Syntheseschritte.

Nach Umkristallisation einer getrockneten Probe aus Diethylether findet man einen Schmelzpunkt von 59-60°C. Der $R_f$ liegt unter den in Beispiel 1 beschriebenen Bedigungen ebenfalls bei 0,95.

**Patentansprüche**

1. Verfahren zur Herstellung von 2-Chlor-5-nitro- und 4-Chlor-3-nitro-benzolsulfonsäurechlorid, dadurch gekennzeichnet, dass man 2-Chlor-5-nitro- und 4-Chlor-3-nitro-benzolsulfonsäure in Oleum mit Thionylchlorid und/oder Chlorsulfonsäure bei Temperaturen von 40 bis 120°C und Normaldruck umsetzt.

2. Verfahren nach Anspruch 1, dadurch gekennzeichnet, dass man in 4 bis 6%igem Oleum arbeitet.

3. Verfahren nach Ansprüchen 1 und 2, dadurch gekennzeichnet, dass man pro Mol 2-Chlor-5-nitro- und 4-Chlor-3-nitro-benzosulfonsäure 2 bis 10 Mol Thionylchlorid und/oder 5 bis 15 Mol Chlorsulfonsäure einsetzt.

4. Verfahren nach Ansprüchen 1 bis 3, dadurch gekennzeichnet, dass man nacheinander 2 bis 10 Mol, vorzugsweise 4 bis 6 Mol Chlorsulfonsäure und 1 bis 5 Mol, vorzugsweise 1 bis 3 Mol Thionylchlorid pro Mol 2-Chlor-5-nitro- und 4-Chlor-3-nitro-benzolsulfonsäure zur Anwendung bringt.

5. Verfahren nach Anspruch 1, dadurch gekennzeichnet, dass man als Eingangsprodukt Reaktionsgemische aus der Sulfierung von o- bzw. p-Nitrochlorbenzol mit Oleum verwendet.

**Claims**

1. Process for the preparation of 2-chloro-5-nitrobenzenesulphonyl chloride and 4-chloro-3-ni- trobenzenesulphonyl chloride, characterized in that 2-chloro-5-nitrobenzenesulphonic acid and 4-chloro-3-nitrobenzenesulphonic acid are reacted in oleum with thionyl chloride and/or chlorosulphonic acid at temperatures of 40 to 120°C, and under normal pressure.

2. Process according to Claim 1, characterized in that the reaction is carried out in 4 to 6% strength oleum.

3. Process according to Claims 1 and 2, characterised in that 2 to 10 moles of thionyl chloride and/or 5 to 15 moles of chlorosulphonic acid are employed per mole of 2-chloro-5-nitrobenzenesulphonic acid and 4-chloro-3-nitrobenzenesulphonic acid.

4. Process according to Claims 1 to 3, characterized in that 2 to 10 moles, preferably 4 to 6 moles, of chlorosulphonic acid and 1 to 5 moles, preferably 1 to 3 moles, of thionyl chloride per mole of 2-chloro-5-nitrobenzenesulphonic acid and 4-chloro-3-nitrobenzenesulphonic acid are used successively.

5. Process according to Claim 1, characterized in that the starting materials used are reaction mixtures from the sulphonation of o-chloronitrobenzene or p-chloronitrobenzene with oleum.

**Revendications**

1. Procédé pour la fabrication de chlorure de 2-chloro-5-nitrobenzènesulfonyle et de chlorure de 4-chloro-3-nitrobenzènesulfonyle, caractérisé en ce que l'on fait réagir l'acide 2-chloro-5-nitrobenzènesulfonique et l'acide 4-chloro-3-nitrobenzènesulfonique dans l'oléum avec le chlorure de thionyle et/ou l'acide chlorosulfonique à des températures de 40 à 120°C et sous pression normale.

2. Procédé selon la revendication 1, caractérisé en ce que l'on opère dans l'oléum à 4-6%.

3. Procédé selon les revendications 1 et 2, caractérisé en ce que l'on utilise 2 à 10 mol de chlorure de thionyle et/ou 5 à 15 mol d'acide chlorosulfonique par mole d'acide 2-chloro-5-nitrobenzènesulfonique et d'acide 4-chloro-3-nitrobenzènesulfonique.

4. Procédé selon les revendications 1 à 3, caractérisé en ce que l'on fait réagir successivement avec 2 à 10 mol, de préférence 4 à 6 mol, d'acide chlorosulfonique et 1 à 5 mol, de préférence 1 à 3 mol, de chlorure de thionyle par mole d'acide 2-chloro-5-nitrobenzènesulfonique et d'acide 4-chloro-3-nitrobenzènesulfonique.

5. Procède selon la revendication 1, caractérisé en ce que l'on utilise comme produit de départ des mélanges de réaction de la sulfonation du o-nitrochlorobenzène et du p-nitrochlorobenzène, respectivement, avec l'oléum.